# EUROPÄISCHE PATENTSCHRIFT

(11) **EP 1 605 891 B1**
(45) Veröffentlichungstag und Bekanntmachung des Hinweises auf die Patenterteilung: **10.09.2008**
(21) Anmeldenummer: 04704190.0
(22) Anmeldetag: 22.01.2004
(51) Int. Cl.: A61J 1/05

(54) **KONNEKTOR FÜR MEDIZINISCHE FLÜSSIGKEITEN ENTHALTENDE VERPACKUNGEN UND VERPACKUNG FÜR MEDIZINISCHE FLÜSSIGKEITEN**
CONNECTOR FOR PACKINGS CONTAINING MEDICAL LIQUIDS, AND CORRESPONDING PACKING FOR MEDICAL LIQUIDS
CONNECTEUR POUR EMBALLAGES CONTENANT DES LIQUIDES MEDICAUX ET EMBALLAGE POUR LIQUIDES MEDICAUX

(30) Priorität: 27.03.2003 DE 10313760
(43) Veröffentlichungstag der Anmeldung: 21.12.2005
(73) Patentinhaber: Fresenius Kabi Deutschland GmbH, 61352 Bad Homburg v.d.H. (DE)
(72) Erfinder: BRANDERBURGER, Torsten, 61194 Niddatal (DE); HEILMANN, Klaus, 66606 St. Wendel (DE); KNIERBEIN, Bernd, 61267 Neu-Anspach (DE)
(74) Vertreter: Luderschmidt, Schüler & Partner
(86) Internationale Anmeldenummer: PCT/EP2004/000487
(87) Internationale Veröffentlichungsnummer: WO 2004/084793

(56) Entgegenhaltungen:
- WO-A-96/23545
- DE-C- 10 030 474
- US-A- 3 913 882
- US-A- 5 121 996
- US-B1- 6 280 431

## Beschreibung

Die Erfindung bezieht sich auf einen Konnektor für Verpackungen, insbesondere Infusions-, Transfusions- oder Enteralbeutel, die medizinische Flüssigkeiten, insbesondere Infusionslösungen oder enterale oder parenterale Nährlösungen enthalten. Darüber hinaus betrifft die Erfindung eine Verpackung für medizinische Flüssigkeiten, insbesondere einen Infusions-, Transfusions- oder Enteralbeutel, mit mindestens einem derartigen Konnektor.

Die DE-A-197 28 775 beschreibt einen Infusionsbeutel mit einem Zuspritzteil und einem Entnahmeteil. Der Zuspritzteil ist zum Zuführen eines Medikaments mittels einer Injektionsspritze bestimmt, während der Entnahmeteil zur Entnahme der Lösung mittels eines Spike dient. Zuspritz- und Entnahmeteil weisen einen rohrförmigen Anschlußteil auf, der von einer als Abbrechteil ausgebildeten Schutzkappe verschlossen ist.

Zuspritz- und Entnahmeteil werden mittels eines Schlauchstücks mit dem Infusions- oder Transfusionsbeutel verbunden. Zum Befüllen des Beutels mit Nährlösung wird in einer Füllstation ein Fülldom in das aus dem Beutel vorstehende Schlauchstück eingeführt und der Beutel mit Nährlösung befüllt. Anschließend wird das Schlauchstück abgequetscht und der Beutel einer Konnektor-Steckstation zugeführt. Dort wird der Zuspritz- bzw. Entnahmeteil auf das Schlauchstück aufgesteckt, und Schlauchstück und Zuspritz- bzw. Entnahmeteil werden miteinander verschweißt. Als nachteilig erweist sich, dass bei der Fertigung des Beutels ein zusätzliches Schlauchstück erforderlich ist. Darüber hinaus ist nachteilig, dass das Befüllen des Beutels und das Anschließen des Zuspritz- bzw. Entnahmeteils in unterschiedlichen Stationen erfolgt.

Der Erfindung liegt daher die Aufgabe zugrunde, einen Konnektor für medizinische Flüssigkeiten enthaltende Verpackungen, insbesondere Infusions-, Transfusions- oder Enteralbeutel, zu schaffen, der die Fertigung des Beutels vereinfacht. Die Lösung dieser Aufgabe erfolgt mit den im Patentanspruch 1 angegebenen Merkmalen.

Darüber hinaus ist eine Aufgabe der Erfindung, eine Verpackung für medizinische Flüssigkeiten, insbesondere Infusions-, Transfusions- oder Enteralbeutel, anzugeben, die sich einfach fertigen lässt. Diese Aufgabe wird mit den im Patentanspruch 10 angegebenen Merkmalen gelöst.

Der erfindungsgemäße Konnektor für medizinische Flüssigkeiten enthaltende Verpackungen, insbesondere Infusions-, Transfusions- oder Enteralbeutel, macht es möglich, in einer Station sowohl die Verpackung zu befüllen als auch den Konnektor anzubringen. Von Vorteil ist, dass ein zusätzliches Schlauchstück zum Verbinden von Konnektor und Verpackung nicht erforderlich ist. Damit entfällt das Schneiden des Schlauchstücks sowie das aufwendige Positionieren.

Der erfindungsgemäße Konnektor verfügt über einen Anschlußteil, der einen Durchgang zur Aufnahme eines Spike zur Entnahme der Flüssigkeit aufweist, und einen auf den Anschlußteil aufsetzbaren Verschlußteil, der den Durchgang in dem Anschlußteil verschließt. Von entscheidender Bedeutung ist, dass die Verpackung durch den Anschlußteil befüllt werden kann. Erst nach dem Befüllen der Verpackung kann der Verschlußteil auf den Anschlußteil aufgesetzt werden.

Der Anschlußteil weist einen elastisch verformbaren Abklemmteil auf, der als rohrförmiger Abschnitt mit einem von der Kreisform abweichenden Querschnitt ausgebildet ist. Nach dem Befüllen der Verpackung kann der Anschlußteil mit einer geeigneten Klemmvorrichtung abgeklemmt werden, so dass beim Aufsetzen des Verschlußteiles nicht die Gefahr des Austretens von Flüssigkeit aus der Verpackung besteht.

Der elastisch verformbare Abklemmteil erlaubt das Befüllen der Verpackung auch im Liegen, was insbesondere bei großvolumigen Beuteln von Vorteil ist. Da der Querschnitt des Abklemmteils von der Kreisform abweicht, kann der Abklemmteil unabhängig von dem verwendeten Material mit verhältnismäßig geringen Kräften abgeklemmt werden. Entscheidend ist, dass der Abklemmteil elastisch verformbar ist, so dass er nach dem Abklemmen wieder seine ursprüngliche Form einnimmt. Dadurch lässt sich eine unerwünschte Querschnittsverringerung des Durchgangs im Anschlußteil vermeiden.

Darüber hinaus macht der Abklemmteil es möglich, den Konnektor bei der Anwendung im Krankenhaus mit einer Klemme oder dgl. zu verschließen.

An dem Abklemmteil schließt sich ein zu beiden Seiten erweiternder Basisteil an, der in die Verpackung integrierbar, vorzugsweise mit dem Beutel verschweißbar ist.

Zur Verringerung des Montageaufwands sind Verschluß- und Anschlußteil des erfindungsgemäßen Konnektors vorzugsweise einschnappend festgelegt. Die Schnappverbindung ermöglicht das Aufsetzen von verschiedenen Verschlußteilen, die in Abhängigkeit vom Anwendungsfall (Infusionen, Transfusionen oder enterale Ernährung) unterschiedlich ausgebildet sein können. Beide Teile können aber auch miteinander verschweißt oder verklebt werden. Auch ist neben dem Formschluß eine zusätzliche Schweißverbindung möglich.

Bei einer weiteren bevorzugten Ausführungsform ist zwischen dem Anschlußteil und dem Verschlußteil eine selbstabdichtende Membran angeordnet, die das Auslaufen der Flüssigkeit aus der Verpackung nach dem Herausziehen des Spike verhindert.

Die Membran wird erst nach dem Befüllen der Verpackung zwischen Anschlußund Verschlußteil eingesetzt. Vorzugsweise wird die Membran zwischen Anschluß- und Verschlußteil unter elastischer Verformung derselben klemmend gehalten. Folglich kann die Montage des Konnektors auf einfache Weise durch das Verpressen der Einzelteile erfolgen. Da die Teile klemmend gehalten werden, besteht nicht die Gefahr der Beschädigung der Membran beim Verschweißen.

Eine weitere bevorzugte Ausführungsform sieht einen Verschlußteil vor, der einen kappenförmigen Unterteil aufweist, an den sich über eine Ringbruchzone ein abbrechbarer Oberteil anschließt. Dieser bildet gleichsam einen Originalitätsverschluß.

Vorzugsweise ist das abbrechbare Oberteil als flaches Griffstück ausgebildet, das sich einfach mit Daumen und Zeigefinger greifen lässt.

Zur Kennzeichnung der Flußrichtung, d. h. ob es sich um einen Zuspritz- oder Entnahmeteil handelt, ist der kappenförmige Unterteil und/oder das flache Griffstück des Verschlußteils vorzugsweise mit einem die Flußrichtung anzeigenden Pfeil gekennzeichnet. Der Pfeil ist vorzugsweise als Aussparung und/oder als erhabene Struktur ausgebildet.

Der in die Verpackung integrierbare Basisteil ist vorzugsweise in der Art eines Schiffchens ausgebildet, das sich mit den Folienlagen eines Beutels einfacher als ein schlauchförmiger Körper verschweißen läßt. Ein derartiges Schiffchen hat bei Konnektoren inzwischen eine Breite Anwendung gefunden.

Die erfindungsgemäße Verpackung für medizinische Flüssigkeiten, insbesondere Infusions-, Transfusions- oder Enteralbeutel, weist mindestens einen Konnektor, vorzugweise zwei Konnektoren auf, von denen der eine als Zuspritzteil und der andere als Entnahmeteil ausgebildet ist.

Nachfolgend wird ein Ausführungsbeispiel der Erfindung unter Bezugnahme auf die Zeichnungen näher erläutert.

Es zeigen:
- Figur 1: einen als Entnahmeteil ausgebildeten Konnektor für einen Nährlösungsbeutel in geschnittener Darstellung,
- Figur 2: den Konnektor von Figur 1 aus der Ansicht des Pfeils II,
- Figur 3: den Verschlußteil des Konnektors von Figur 1 in der Seitenansicht,
- Figur 4: eine Ansicht des Verschlußteils von Fig.3 aus der Ansicht des Pfeils V
- Figur 5a + 5b: zwei unterschiedliche Querschnittsformen des elastisch verformbaren Abklemmteils und
- Figur 6a +6b: zwei alternative Ausführungsformen des Abklemmteils und
- Fig. 7: einen Folienbeutel für parenterale Nährlösung mit einem Zuspritz-und Entnahmeteil.

Der als Entnahmeteil ausgebildete Konnektor A beispielsweise für parenterale Nährlösungsbeutel verfügt über einen Anschlußteil 1, der einen Durchgang 2 zur Aufnahme eines nicht dargestellten Spike zur Entnahme der Flüssigkeit aufweist. Auf den Anschlußteil 1 ist ein Verschlußteil 3 aufgesetzt, der den Durchgang 2 in dem Anschlußteil 1 verschließt. Anschlußteil und Verschlußteil sind Spritzgießteile aus Polypropylen.

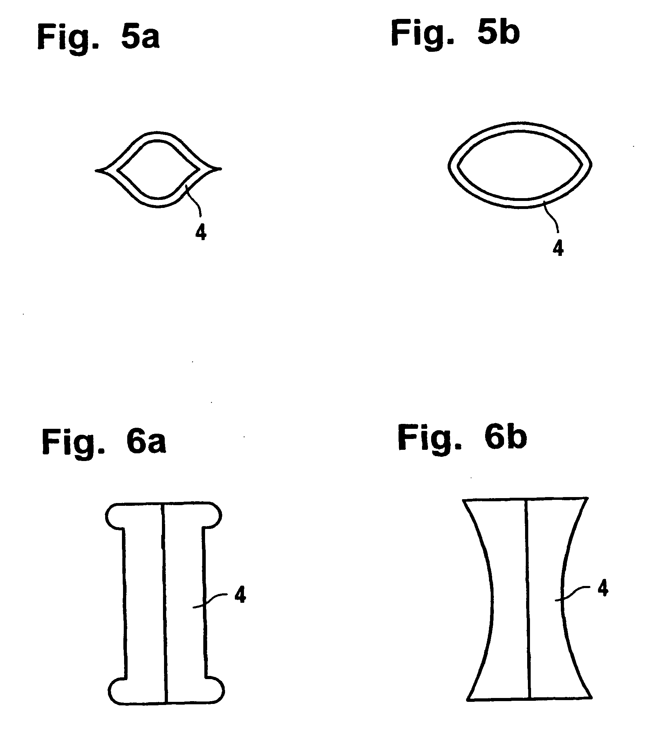

Der Anschlußteil 1 weist einen elastisch verformbaren Abklemmteil 4 auf, der mit einem von der Kreisform abweichenden Querschnitt ausgebildet ist. Die Figuren 5a und 5b zeigen alternative Querschnittsformen des Abklemmteils 4. Allen Querschnittsformen ist gemeinsam, dass die Ausdehnung des Abklemmteils in zwei senkrecht aufeinander stehenden Richtungen unterschiedlich ist. Dadurch wird erreicht, dass sich der Abklemmteil mit verhältnismäßig geringen Kräften zusammendrücken läßt. Entscheidend ist, dass der Abklemmteil beim Zusammendrücken nicht bricht.

Der Abklemmteil kann in Längsrichtung unterschiedlich ausgebildet sein. Beispielsweise ist eine gleichbleibende Querschnittsverringerung in Längsrichtung möglich. Auch kann der Abklemmteil mit einer Einschnürung versehen sein. Diese Alternativen zeigen die Figuren 6a und 6b.

An den Abklemmteil 4 schließt sich ein zu beiden Seiten erweiterndes Basisteil 5 an, das nach Art eines Schiffchens mit spitz auslaufenden Seitenstücken ausgebildet ist. Ober- und Unterseite des Schiffchens können mit den Innenseiten der oberen und unteren Folienlage des Beutels einfach verschweißt werden.

Der Verschlußteil 3 weist ein kappenförmiges Unterteil 6 auf, an das sich über eine Ringbruchzone 7 ein abbrechbares Oberteil 8 anschließt. Das kappenförmige Unterteil 6 des Verschlußteils 3 ist einschnappend an einem zylindrischen Ansatz des Verschlußteils 1 festgelegt. Die Innenwand des Unterteils 6 weist eine umlaufende Nut 9 auf, in die ein umlaufender Vorsprung 10 an der Außenwand des zylindrischen Ansatzes des Verschlußteils 1 beim Zusammenpressen der beiden Teilstücke 1,3 einschnappt.

Zwischen dem Verschlußteil 3 und dem Anschlußteil 1 wird eine selbstabdichtende Membran 11, die auch als Septum bezeichnet wird, unter elastischer Verformung derselben klemmend gehalten. Die selbstabdichtende Membran 11 weist einen äußeren Abschnitt 12 auf, der zwischen dem zylindrischen Ansatz des Anschlußstücks 1 und dem Unterteil 6 des Verschlußteils 3 verklemmt ist. An den äußeren Abschnitt 12 schließt sich ein oberer ringförmiger Abschnitt 13 an, der unter Bildung einer muldenförmigen Vertiefung 14 an der Oberseite der Membran in einen unteren tellerförmigen Abschnitt 15 übergeht. Im Zentrum ist der tellerförmige Abschnitt 15 kreuzweise oder sternförmig vorgeschlitzt, so dass das elastische Material zwar geschwächt, aber nicht durchtrennt ist.

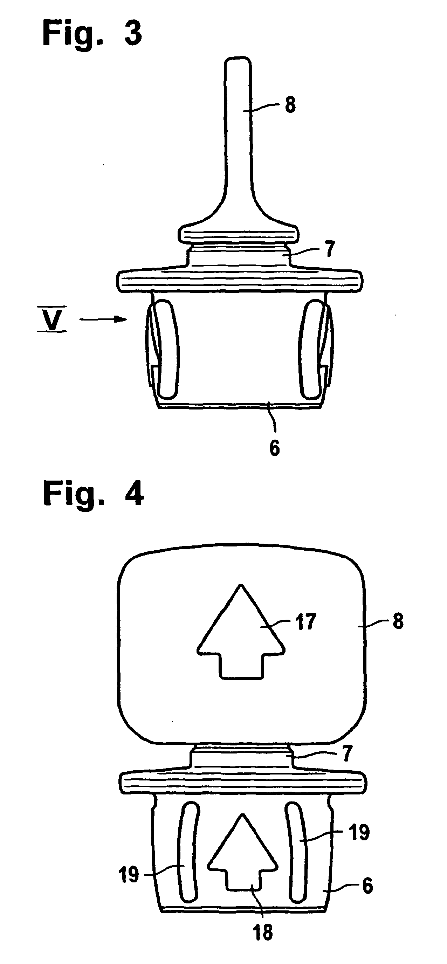

Der Griffteil 8 des Verschlußteils 3, der die obere Öffnung 16 zur Entnahme der Nährlösung verschließt, ist als flacher Körper ausgebildet, der mit Daumen und Zeigefinger leicht gegriffen und abgedreht werden kann. Im Zentrum des Griffstücks befindet sich eine Aussparung 17 in Pfeilform. An zwei gegenüberliegenden Seiten des Unterteils 6 des Verschlußteils 3 befindet sich ebenfalls ein Pfeil 18, der allerdings als erhabene Struktur ausgebildet und zwischen zwei eine Griffmulde bildende Begrenzungen 19 angeordnet ist (Figuren 3 und 4).

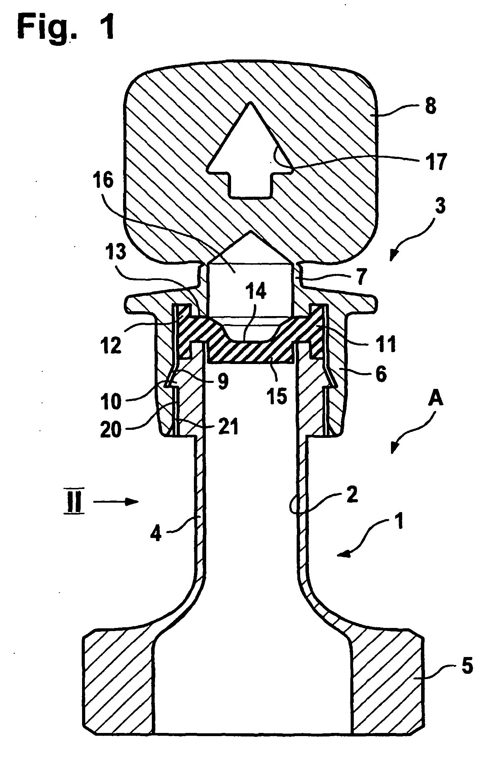

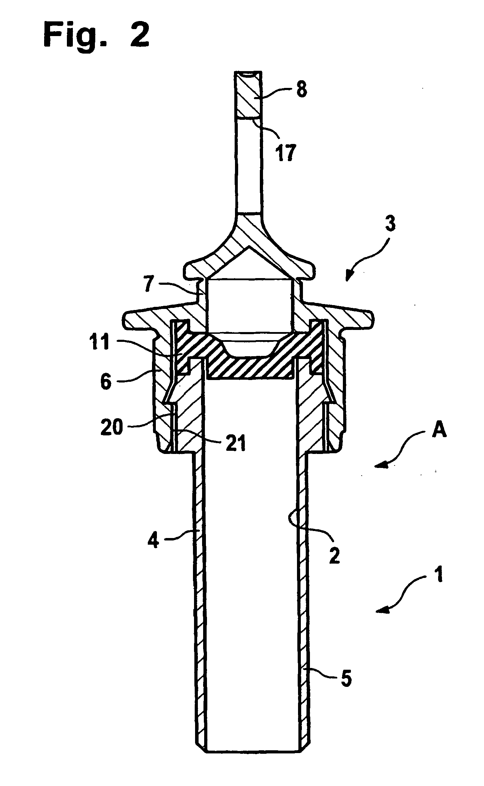

Zur Sicherung des Verschlußteils 3 und des Anschlußteils 1 gegen Verdrehen sind an der Innenseite des kappenförmigen Unterteils 6 und der Außenseite des zylindrischen Ansatzes des Entnahmeteils 1 eine keilwellenförmige Innen- bzw. Außenverzahnung 20, 21 vorgesehen (Figuren 1 und 2). Mit der keilwellenförmigen Verzahnung lassen sich die Teile beim Fügen exakt gegeneinander ausrichten.

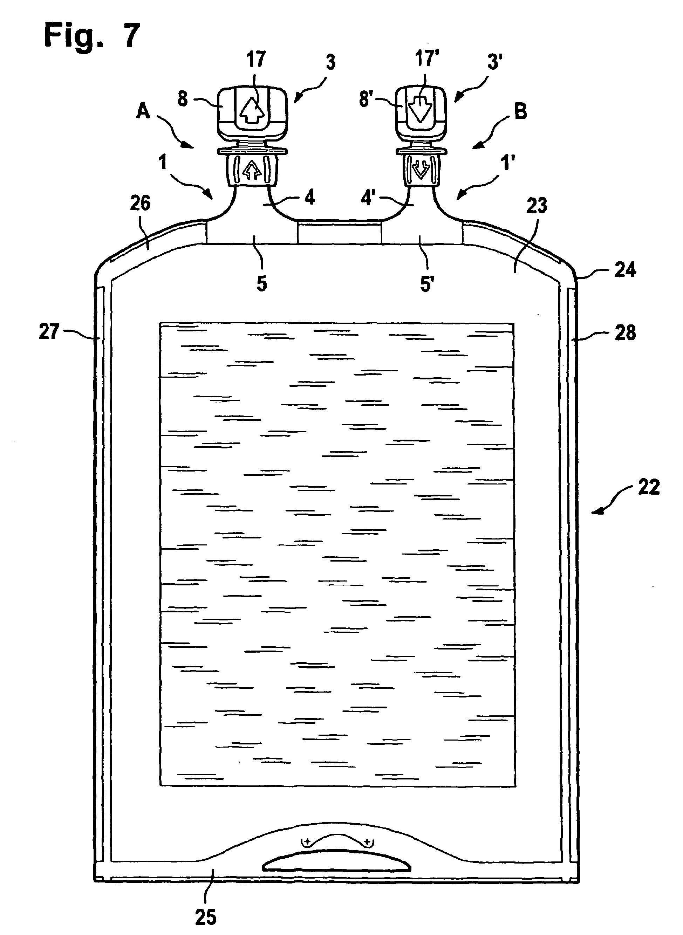

Figur 7 zeigt einen mit einer Nährlösung für die parenterale Ernährung befüllten Folienbeutel 22, der über den Konnektor A zur Entnahme der Nährlösung und einen weiteren Konnektor B zum Zuspritzen einer Lösung in den Infusionsbeutel verfügt. Bei dem Konnektor A handelt es sich um einen Zuspritzteil, der den gleichen Aufbau wie der Entnahmeteil hat. Er verfügt wieder über einen Anschlußteil 1' und einen Verschlußteil 3'. Die entgegengesetzte Flußrichtung ist mit dem Pfeil 17' gekennzeichnet. Dadurch lassen sich Entnahme- und Zuspritzteil einfach voneinander unterscheiden.

Der Folienbeutel 22 besteht aus zwei Folienlagen 23, 24, die am unteren und oberen Rand 25, 26 sowie den längslaufenden Rändern 27, 28 miteinander verschweißt sind. Zwischen die beiden Folienlagen 23, 24 sind am oberen Rand des Beutels die in der Art eines Schiffchens ausgebildeten Basisteile 5, 5' der Konnektoren A, B eingeschweißt.

Bei der Fertigung wird der Beutel mit einem Fülldom befüllt, der in das Anschlußteil 1,1' einer der beiden Konnektoren eingeführt wird, bevor der Verschlußteil 3, 3' auf den Anschlußteil aufgesetzt wird. Anschließend wird der Abklemmteil 4, 4' mit einer geeigneten Abklemmeinrichtung, die beispielsweise über zwei gegeneinander verschiebbare Druckelemente verfügen kann, abgeklemmt, so dass Flüssigkeit nicht entweichen kann. Anschließend wird der Verschlußteil 3 auf den Anschlußteil 1 gepreßt, so dass beide Teile einschnappend festgelegt sind. Damit ist der Konnektor A verschlossen. Zum Entnehmen der Nährlösung wird der Abbrechteil 8 des Verschlußteils 3 durch Drehen oder Brechen desselben abgebrochen, so dass die selbstabdichtende Membran 11 frei liegt. In die Öffnung 16 des Anschlußteils 1 wird nun der Spike eines bekannten Überleitsystems eingeschoben, wodurch die vorgeschlitzte Membran 11 durchstochen wird. Dabei dient die muldenförmige Vertiefung 14 der Membran 11 als Führung für den Spike. Der Spike wird mit dem ringförmigen Abschnitt 13 der Membran abgedichtet. Aufgrund der besonderen Ausbildung der Membran wird der Spike fest in dem Anschlußteil 1 gehalten.

Darauf hin kann die parenterale Nährlösung entnommen werden. Wenn der Spike wieder herausgezogen ist, dichtet die Membran den Anschlußteil auch bei einem relativ hohen Innendruck sicher ab. Im übrigen wird mit der besonderen Ausbildung der Membran die mechanische Festigkeit des Anschlußteils erhöht.

Der Zuspritzteil B dient dazu, der Lösung eine Wirksubstanz zuspritzen zu können. Hierzu wird wieder nach Entfernen des Abbrechteils 8' die Membran mit einer Injektionsnadel einer Spritze durchstochen. Nach Herausziehen der Nadel dichtet der Zuspritzteil wieder ab. Enterale Nährlösungsbeutel besitzen hingegen üblicherweise keinen Zuspritzteil, sondern nur einen Entnahmeteil.

## Patentansprüche

1. Konnektor für medizinische Flüssigkeiten enthaltende Verpackungen, insbesondere Infusions-, Transfusions- oder Enteralbeutel, mit
einem Anschlußteil (1), der einen Durchgang (2) zur Aufnahme eines Spike zur Entnahme der Flüssigkeit aufweist,
einem auf den Anschlußteil (1) aufsetzbaren Verschlußteil (3), der den Durchgang (2) in dem Anschlußteil verschließt,
**dadurch gekennzeichnet,**
**dass** der Anschlußteil (1) einen elastisch verformbaren Abklemmteil (4) aufweist, der als rohrförmiger Abschnitt mit einem von der Kreisform abweichenden Querschnitt ausgebildet ist, und dass der Abklemmteil (4) in einen sich zu beiden Seiten erweiternden Basisteil (5) übergeht, der in die Verpackung integrierbar ist.

2. Konnektor nach Anspruch 1, **dadurch gekennzeichnet, dass** der Verschlußteil (3) und der Anschlußteil (1) einschnappend festgelegt sind.

3. Konnektor nach Anspruch 1 oder 2, **dadurch gekennzeichnet, dass** zwischen dem Anschlußteil (1) und dem Verschlußteil (3) eine selbstabdichtende Membran (11) angeordnet ist, die von dem Spike zur Entnahme der Flüssigkeit durchstechbar ist.

4. Konnektor nach Anspruch 3, **dadurch gekennzeichnet, dass** die selbstabdichtende Membran (11) zwischen dem Anschlußteil (1) und dem Verschlußteil (3) unter elastischer Verformung derselben klemmend gehalten wird.

5. Konnektor nach einem der Ansprüche 1 bis 4, **dadurch gekennzeichnet, dass** der Verschlußteil (3) ein kappenförmiges Unterteil (6) aufweist, an den sich über eine Ringbruchzone (7) ein abbrechbares Oberteil (8) anschließt.

6. Konnektor nach Anspruch 5, **dadurch gekennzeichnet, dass** das abbrechbare Oberteil (8) als flaches Griffstück ausgebildet ist.

7. Konnektor nach Anspruch 6, **dadurch gekennzeichnet, dass** das kappenförmige Unterteil (6) und/oder das flache Griffstück (8) mit einem die Flußrichtung anzeigenden Pfeil (17,18) **gekennzeichnet** ist.

8. Konnektor nach Anspruch 7, **dadurch gekennzeichnet, dass** der Pfeil (17, 18) als Aussparung und/oder als erhabene Struktur ausgebildet ist.

9. Konnektor nach einem der Ansprüche 1 bis 8, **dadurch gekennzeichnet, dass** der Basisteil (5) in der Art eines Schiffchens ausgebildet ist.

10. Verpackung für medizinische Flüssigkeiten, insbesondere Infusions-, Transfusions- oder Enteralbeutel, mit mindestens einem Konnektor nach einem der Ansprüche 1 bis 9.

## Claims

1. Connector for packs containing medical liquids and in particular infusion, transfusion or enteral bags, having
a connecting portion (1) which has passing through it a passage (2) to receive a spike for the withdrawal of the liquid,
a closure portion (3) which can be fitted onto the connecting portion (1) and which closes off the through-passage (2) in the connecting portion,
**characterised in that**
the connecting portion (1) has an elastically deformable clamping-off portion (4) which is in the form of a tubular section of non-circular cross-section, and **in that** the clamping-off portion (4) merges into a base portion (5) which widens out on both sides and which can be incorporated into the pack.

2. Connector according to claim 1, **characterised in that** the closure portion (3) and connecting portion (1) are fixed together by snapping on.

3. Connector according to claim 1 or 2, **characterised in that** a self-sealing membrane (11), which can be pierced by the spike to allow the liquid to be withdrawn, is arranged between the connecting portion (1) and the closure portion (3).

4. Connector according to claim 3, **characterised in that** the self-sealing membrane (11) is held between the connecting portion (1) and closure portion (3) by a clamping action resulting from the elastic deformation of the two latter.

5. Connector according to one of claims 1 to 4, **characterised in that** the closure portion (3) has a cap-like bottom part (6) which continues, via an annular fracturable zone (7), into a top part (8) which can be broken off.

6. Connector according to claim 5, **characterised in that** the top part (8) able to be broken off takes the form of a flat grippable part.

7. Connector according to claim 6, **characterised in that** the cap-like bottom part (6) and/or the flat grippable part (8) is marked with an arrow (17, 18) indicating the direction of flow.

8. Connector according to claim 7, **characterised in that** the arrow (17, 18) takes the form of a recess and/or a raised structure.

9. Connector according to one of claims 1 to 8, **characterised in that** the base portion (5) is formed to resemble a boat.

10. Pack for medical liquids, and in particular an infusion, transfusion or enteral bag, having at least one connector according to one of claims 1 to 9.

## Revendications

1. Connecteur pour emballages contenant des liquides médicaux, en particulier des poches de perfusion, de transfusion ou de nutrition entérale, comportant
un élément de raccordement (1), qui présente un passage (2) destiné à recevoir une aiguille permettant le prélèvement du liquide,
un élément d'obturation (3) pouvant être monté sur l'élément de raccordement (1), qui obture le passage (2) dans l'élément de raccordement,
**caractérisé en ce**
**que** l'élément de raccordement (1) comprend un élément de pincement (4) élastiquement déformable, qui est réalisé sous forme de partie tubulaire avec une section transversale divergeant de la forme circulaire, et en ce que l'élément de pincement (4) se transforme en un élément de base (5) s'élargissant vers les deux côtés, qui peut être intégré dans l'emballage.

2. Connecteur selon la revendication 1, **caractérisé en ce que** l'élément d'obturation (3) et l'élément de raccordement (1) sont fixés par encliquetage.

3. Connecteur selon la revendication 1 ou 2, **caractérisé en ce qu'**une membrane autoétanchéifiante (11), qui peut être percée par l'aiguille permettant le prélèvement du liquide est disposée entre l'élément de raccordement (1) et l'élément d'obturation (3).

4. Connecteur selon la revendication 3, **caractérisé en ce que** la membrane autoétanchéifiante (11) est maintenue serrée par déformation élastique de celle-ci entre l'élément de raccordement (1) et l'élément d'obturation (3).

5. Connecteur selon l'une quelconque des revendications 1 à 4, **caractérisé en ce que** l'élément d'obturation (3) présente un élément inférieur (6) en forme de capuchon, au niveau duquel se raccorde un élément supérieur (8) sécable par le biais d'une zone de rupture annulaire (7).

6. Connecteur selon la revendication 5, **caractérisé en ce que** l'élément supérieur (8) sécable est réalisé sous forme d'élément de préhension plat.

7. Connecteur selon la revendication 6, **caractérisé en ce que** l'élément inférieur (6) en forme de capuchon et/ou l'élément de préhension (8) plat est repéré par une flèche (17, 18) indiquant le sens d'écoulement.

8. Connecteur selon la revendication 7, **caractérisé en ce que** la flèche (17, 18) est réalisée sous forme d'évidement et/ou de structure en relief.

9. Connecteur selon l'une quelconque des revendications 1 à 8, **caractérisé en ce que** l'élément de base (5) est réalisé à la manière d'un petit bateau.

10. Emballage pour liquides médicaux, en particulier poche de perfusion, de transfusion ou de nutrition entérale, avec au moins un connecteur selon l'une quelconque des revendications 1 à 9.
